(19) Europäisches Patentamt
European Patent Office
Office européen des brevets

(11) Numéro de publication : **0 334 776 B1**

(12) # FASCICULE DE BREVET EUROPEEN

(45) Date de publication du fascicule du brevet : **31.07.91 Bulletin 91/31**

(51) Int. Cl.$^5$ : **A61K 35/20**, A61K 7/48, A61K 7/08

(21) Numéro de dépôt : **89420093.0**

(22) Date de dépôt : **16.03.89**

(54) **Composition cosmétique ou pharmaceutique à usage cutané, et procédé pour la préparation d'une telle composition.**

(30) Priorité : **25.03.88 FR 8804238**

(43) Date de publication de la demande : **27.09.89 Bulletin 89/39**

(45) Mention de la délivrance du brevet : **31.07.91 Bulletin 91/31**

(84) Etats contractants désignés : **BE CH DE ES FR GB IT LI NL**

(56) Documents cités : **FR-A- 2 460 135**

(73) Titulaire : **GATTEFOSSE S.A.**
**36 chemin de Genas**
**F-69800 Saint Priest (FR)**

(72) Inventeur : **Garcin, Isabelle**
**25 rue Guilloud**
**F-69003 Lyon (FR)**
Inventeur : **Paviot, Gisèle**
**254 rue de Créqui**
**F-69003 Lyon (FR)**

(74) Mandataire : **Laurent, Michel et al**
**Cabinet LAURENT et CHARRAS, 20, rue Louis Chirpaz B.P. 32**
**F-69131 Ecully Cedex (FR)**

**EP 0 334 776 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

**Description**

L'invention concerne une nouvelle composition cosmétique ou pharmaceutique à usage cutané ; elle se rapporte également à un procédé pour la fabrication d'une telle composition.

Les nouvelles compositions cosmétiques ou pharmaceutiques à usage cutané selon l'invention se caractérisent en ce qu'elles contiennent comme principe actif du sérum de colostrum.

Comme on le sait, le "colostrum" est le lait (liquide jaunâtre et opaque) sécrété par la glande mammaire durant les derniers temps de la grossesse et plus particulièrement les tous premiers jours qui suivent l'accouchement. Le "sérum" est la partie liquide qui se sépare de ce lait après coagulation, acide ou enzymatique. Le "sérum de colostrum" est donc la fraction du colostrum qui a été débarrassé de ses matières grasses et des protéines caséïniques.

La nouvelle composition à usage externe (cutané) selon l'invention contient donc du sérum de colostrum comme principe actif et des ingrédients d'usage courant pour l'application envisagée.

On a déjà proposé d'utiliser le colostrum dans la fabrication des collyres (voir par exemple document FR-A-2460135 ou son correspondant US-A-4342747). L'invention concerne non pas une utilisation du colostrum, mais d'un de ses constituants, à savoir : le sérum, et non pas son application comme collyres, mais comme composant d'une composition à application cutanée pour l'industrie des cosmétiques ou pharmaceutique.

Jusqu'à ce jour, on ne sait pas isoler industriellement la phase sérum du colostrum. Les techniques fromagères usuelles de caillage sont totalement inefficaces. C'est la raison sans doute pour laquelle le colostrum n'a guère été étudié et n'a pratiquement été exploité que pour l'alimentation des veaux. Les autres techniques de séparation connues dans l'industrie fromagère telles que la centrifugation, la centrifugation à grande vitesse, la filtration, la filtration sur membrane, la clarification, ne donnent aucun résultat exploitable lorsque on les applique au colostrum.

Comme on le sait, la "microfiltration" est une technique de séparation par diffusion sous pression d'un liquide envoyé de préférence de manière tangentielle, sur une membrane perméable. Cette technique se différencie essentiellement de l'osmose inverse et/ou de l'ultrafiltration par le seuil de coupure des membranes utilisées.

Dans le procédé selon l'invention, avantageusement :

— le colostrum est un colostrum bovin issu des trois premières traites après le velage ; on a constaté que dans les sérums séparés à partir des colostrums des traites suivantes, le taux de facteurs de croissance diminuait rapidement, ce qui réduit considérablement l'intérêt économique de ce sérum pour le domaine d'application envisagé ;

— la microfiltration est effectuée à une température inférieure à 50°C et de préférence à température ambiante, sur une membrane minérale de la troisième génération dont les pores ont une dimension inférieure à 0,15 micromètre, de préférence voisine de 0,14 micromètre ; en effet, on a observé que si la température dépassait 50°C, on risque de dénaturer le colostrum alors que si les pores ont une dimension inférieure à 0,15 micromètre, on risque d'éliminer des composés recherchés pour les applications cutanées visées ;

— le sérum de colostrum ainsi séparé est introduit dans une formulation de composition cosmétique ou pharmaceutique à usage cutané, à raison d'au moins 1% en poids du poids de ladite composition, de préférence de l'ordre d'au moins 2,5%, avantageusement inférieure à 10% ; en effet, on a observé qu'au-delà de 10% en poids, on n'observe pas d'amélioration proportionnelle de sorte que cette technique perd de l'intérêt sur le plan économique ;

— le sérum de colostrum ainsi séparé se présente sous forme d'un liquide jaunâtre limpide contenant essentiellement :

- alpha lactalalbulmine,
- bêta lactoglobuline,
- des immoglobulines,
- des facteurs de croissance épidermique, BCGF (Bovin colostrum growth factor)
- des vitamines,
- des oligo-éléments,
- du lactose,
- des protéines solubles ;

— ce sérum de colostrum ainsi séparé n'est pas toxique et peut donc concerner toutes les applications cutanées tant dans le domaine de la pharmacie que de celui de la cosmétologie ; ce composé peut être utilisé en cure soit comme déjà dit, de préférence comme base dans des formulations habituelles ;

— ce sérum peut être associé en proportions appropriées (1 à 10%) dans une formulation avec d'autres substances actives d'origine biologique telles que le collagène, l'acide desoxyribolucleique, les glycosaminoglycanes ou autres (albumine, keratine, extraits placentaires, liquide amniotique...).

Les compositions ainsi réalisées peuvent également être utilisées comme pansement cutané ou même comme prothèse de peau. Le plus avantageusement, ces compositions sont utilisées comme produits d'hygiène corporelle à but cosmétique ou pharmaceutique, notamment à visée hydratante, stimulante, adoucissante, nourrissante, régénératrice, cicatrisante.

La manière dont l'invention peut être réalisée et les avantages qui en découlent ressortiront mieux des exemples de réalisation qui suivent, donnés à titre indicatif et non limitatif.

## Exemple 1

On écrème à 40°C vingt kilos de colostrum bovin frais issus des trois premières traites après le velage. Ce colostrum a un pH de 5,3.

On microfiltre ce colostrum chauffé à une température comprise entre 35 et 40°C par passage sur une membrane minérale de la troisième génération marque **Carbosep-M14 (de SFEC-Union Carbide)** ayant les caractéristiques suivantes :

- — dimension des pores : 0,14 micromètre,
- — débit de circulation : 1600 l/h,
- — pression moyenne : 0,9 bar,
- — vitesse de balayage : 5,3 m/s,
- — débit moyen du permeat : 21,8 l/m2/h.

On obtient dix-huit kilos d'un rétentat liquide, trouble et deux kilos d'un permeat jaune d'or, limpide, de pH 5,3, stable dans le temps à température ambiante et contenant :

- — matières sèches : 71,5 g/l,
- — matières minérales : 6,5 g/l,
- — % en extraits secs : 9,1,
- — azote total : 4,5 g/l,
- — taux global de protéines : 28,1%,
- — lactose : 30 g/l,
- — vitamines :
  - • $B_1$ : 0,9 mg/l,
  - • $B_2$ : 2 mg/l,
  - • $B_{12}$ : 3,5 mg/l,
  - • $B_5$ : 0,46 mg/l,
  - • PP : 0,8 mg/l,
  - • C : 180 mg/l,
  - • E : 0,06 mg/l,
  - • H : 1 mg/l,
- — BCGF de poids moléculaire voisin de 6000 d, détecté par la mesure de la croissance cellulaire sur milieu non enrichi ;

L'électrophorese sur acétate de cellulose de ce permeat est normale. Ce composé est totalement exempt de caséïne.

L'analyse détecte :

- — 9% du total des protéines d'alpha lactalbumine,
- — 27,5% de béta lactoglubuline,
- — 60% d'immunoglobuline,
- — 3,5% d'albumines.

Ce composé stocké pendant un an à température ambiante sans précaution particulière est parfaitement stable puisqu'aucun gaz, ni aucune odeur nauséabonde ne se dégage.

Dans une formulation connue pour cosmétologie, on remplace poids pour poids de l'extrait placentaire aqueux par le sérum de colostrum ainsi obtenu. On obtient une crème ayant d'excellentes propriétés régénératrices de la peau, cicatrisantes, et reconstituantes des cellules épidermiques.

Cette composition peut donc être utilisée avantageusement soit comme composition cosmétologique, soit même comme composition pharmaceutique à usage cutané.

## Exemple 2 :

On répète l'exemple 1.

Aux dix-huit kilos de rétentat, on ajoute en permanence de l'eau distillée pour avoir un volume constant (diafiltration).

On microfiltre ce liquide dans les mêmes conditions qu'à l'exemple 1 jusqu'à épuisement du rétentat.

On récupère le perméat et par osmose inverse, on lui fait subir une nouvelle concentration.

On obtient un liquide ayant les mêmes concentrations et les mêmes propriétés qu'à l'exemple 1.

## Exemple 3 :

Le sérum de colostrum obtenu à l'exemple 1 est stérilisé par passage au travers d'un filtre stérilisant avant d'être utilisé dans une formulation.

Ces compositions sont particulièrement adaptées dans l'application pharmaceutique.

## Exemple 4 :

On répète l'exemple 1, mais en remplaçant la membrane minérale par une membrane organique PVDF, c'est-à-dire en polyvinylidène fluoré, ayant une dimension de pres voisine de 0,1 micromètre. De manière surprenante, on recueille une composition identique, mais enrichit et à tout le moins plus pure en facteurs de croissance et en vitamines.

Comme déjà dit, le sérum de colostrum conforme à l'invention peut être utilisé en cure ou encore mieux comme base dans une formulation. Il peut être utilisé soit sous forme de liquide stérile, voire même sous forme solide après lyophilisation. Il peut être utilisé sous des formes les plus diverses telles que films, éponges, formes émulsionnées, gels, lotions, pommades, crèmes, pansements, etc..

Comme application thérapeutique, on peut citer la régénération superficielle de la peau, la cicatrisation, animale ou humaine, la régénération de la peau en cas de brûlure du premier degré.

Dans les applications cosmétologiques, on peut citer les produits d'hygiène corporelles, les shampooings, les crèmes, les lotions, les sticks, les laits hydratants, les produits de maquillage.

## Revendications

1. Composition cosmétique ou pharmaceutique, à usage cutané, caractérisée en ce qu'elle contient comme principe actif du sérum de colostrum.

2. Composition selon la revendication 1, caractérisée en ce qu'elle contient en poids au moins 1%, de préférence entre 2,5 et 10% en poids du sérum de colostrum.

3. Composition selon l'une des revendications 1 et 2, caractérisée en ce qu'elle contient également d'autres substances actives d'origine biologique choisies dans le groupe comprenant le collagène, l'acide désoxyribonucléique, les glycosaminoglycanes, l'albumine, la keratine, les extraits placentaires, le liquide amniotique.

4. Composition selon la revendication 1, caractérisée en ce qu'elle utilisée comme produit d'hygiène corporelle hydratante, nourrissante, génératrice et cicatrisante de la peau.

5. Procédé pour la préparation d'une composition cosmétique ou pharmaceutique à usage cutané selon l'une des revendications 1 à 4, caractérisé en ce qu'il consiste :

— à collecter un colostrum issu des trois premières traites après le velage ;

— puis, à séparer le sérum de ce colostrum par filtration poussée ;

— et enfin, à introduire le sérum de colostrum ainsi séparé dans une formulation de composition cosmétique ou pharmaceutique à usage cutané, à raison d'au moins 1% et au plus 10% en poids du poids de ladite composition.

6. Procédé selon la revendication 5, caractérisé en ce que le colostrum collecté est écrémé, et en ce que la filtration poussée est une microfiltration ou une ultrafiltration.

7. Procédé selon la revendication 6, caractérisé en ce que la microfiltration est effectuée à une température inférieure à 50°C sur une membrane minérale de la troisième génération dont les pores ont une dimension inférieure à 0,15 micromètre.

## Patentansprüche

1. Kosmetische oder pharmazeutische Zusammensetzung zur Anwendung auf der Haut, dadurch gekennzeichnet, daß sie als Wirkstoff Kolostrumserum enthält.

2. Zusammensetzung nach Anspruch 1, dadurch gekennzeichnet, daß sie zumindest 1 Gew.-%, vorzugsweise zwischen 2,5 und 10 Gew.-% an Kolostrumserum enthält.

3. Zusammensetzung nach Anspruch 1 oder 2, dadurch gekennzeichnet, daß sie weitere aktive Substanzen biologischen Ursprungs enthält, ausgewählt aus der Gruppe bestehend aus Kollagen, Desoxyribonucleinsäure, Glykosaminoglykane, Albumin, Keratin, Plazentaextrakte, Fruchtwasser.

4. Zusammensetzung nach Anspruch 1, dadurch gekennzeichnet, daß sie als für die Haut feuchtigkeitsspendendes, nährendes, neubildendes und heilungsförderndes Körperhygienemittel verwendet wird.

5. Verfahren zum Herstellen einer kosmetischen oder pharmazeutischen Zusammensetzung nach einem der Ansprüche 1 bis 4, dadurch gekennzeichnet, daß es die Schritte enthält:

— Sammeln von Kolostrum aus den drei ersten Melkvorgängen nach dem Kalben ;

— anschließend Abbrennen von Kolostrumserum durch Druckfiltration ;

— und schließlich Einbringen des so abgetrennten Kolostrumserums in eine Rezeptur einer kosmetischen oder pharmazeutischen Zusammensetzung zur Anwendung auf der Haut, und zwar mit zumindest 1 Gew.-% und höchstens 10 Gew.-% bezüglich des Gewichtes der Zusammensetzung.

6. Verfahren nach Anspruch 5, dadurch gekennzeichnet, daß das gesammelte Kolostrum entrahmt ist, und daß die Druckfiltration eine Mikrofiltration oder eine Ultrafiltration ist.

7. Verfahren nach Anspruch 6, dadurch gekennzeichnet, daß die Mikrofiltration bei einer Temperatur unterhalb von 50°C über eine mineralische Membran der dritten Generation durchgeführt wird, deren Poren eine Größe unterhalb von 0,15 Mikrometer aufweisen.

## Claims

1. A cosmetic or pharmaceutical composition for cutaneous use, characterized that it contains as active ingredient serum of colostrum.

2. The composition of Claim 1, wherein it contains by weight at least 1%, and preferably between 2.5 and 10% by weight, of serum of colostrum.

3. The composition of one of claims 1 and 2, wherein it also contains other active substances of biological origin, selected from the group comprising collagen, DNA : desoxyribonucleic acid, glycosaminoglycans, albumin, keratin, placenta extrats, amniotic liquid.

4. The composition of Claim 1, wherein it is used as body hygiene product for hydrating, nourishing, generating or healing the skin.

5. A process for preparing the cosmetic or pharmaceutical composition for cutaneous, use, of one of Claims 1 to 4, characterized that it consists in :

— collecting colostrum issuing from the first three milkings after calving ;

— then separating the serum from this colostrum by intense filtration ;

— and finally, introducing the serum of colostrum thus separated into a formulation of cosmetic or pharmaceutical composition for cutaneous use, at a rate of at least 1% and at the most 10% by weight of the weight of said composition.

6. The process of Claim 5, wherein the collected colostrum is skimmed, and the intense filtration is a micro-filtration or an ultra-filtration.

7. The process of Claim 6, wherein micro-filtration is effected at a temperature lower than 50°C on a third-generation inorganic membrane whose pores have a dimension smaller than 0.15 micrometer.